# FASCICULE DE BREVET EUROPEEN

(11) **EP 2 473 517 B1**
(45) Date de publication et mention de la délivrance du brevet: **14.10.2015**
(21) Numéro de dépôt: 10768510.9
(22) Date de dépôt: 31.08.2010
(51) Int. Cl.: C07K 5/10, A61K 38/07, C07K 7/06, A61K 8/64, A61Q 19/08, A61P 17/16

(54) **PEPTIDE ACTIVATEUR DE LA TRANSGLUTAMINASE ET COMPOSITION COSMETIQUE OU PHARMACEUTIQUE LE CONTENANT**
TRANSGLUTAMINASE-AKTIVIERENDES PEPTID UND KOSMETISCHE ODER PHARMAZEUTISCHE ZUSAMMENSETZUNG DAMIT
TRANSGLUTAMINASE-ACTIVATING PEPTIDE, AND COSMETIC OR PHARMACEUTICAL COMPOSITION CONTAINING SAME

(30) Priorité: 04.09.2009 FR 0904218
(43) Date de publication de la demande: 11.07.2012
(73) Titulaire: ISP Investments Inc., Wilmington, DE 19805 (US)
(72) Inventeur: DAL FARRA, Claude, Kerhonkson, NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2010/000595
(87) Numéro de publication internationale: WO 2011/027048

(56) Documents cités:
- WO-A1-97/47314
- WO-A2-97/02340
- WO-A2-2005/054222
- JP-A- 2004 115 451
- US-A1- 2002 076 834
- US-A1- 2006 149 034
- HITOMI KIYOTAKA: "Transglutaminases in skin epidermis.", EUROPEAN JOURNAL OF DERMATOLOGY : EJD 2005 SEP-OCT, vol. 15, no. 5, septembre 2005 (2005-09), pages 313-319, XP002575628, ISSN: 1167-1122

## Description

La présente invention se situe dans le domaine cosmétique et pharmaceutique, et plus particulièrement dans le domaine de la dermatologie. La présente invention concerne des peptides de formule générale (I) :

R₁-(AA)ₙ-X₁-X₂₋-Arg-Arg-Gly-X₃-X₄-(AA)ₚ-R₂.

La présente invention concerne également une composition cosmétique ou pharmaceutique, comprenant un peptide de formule générale (I), utilisé seul ou en association avec au moins un autre principe actif, dans un milieu physiologiquement adapté. L'invention est également relative à l'utilisation de ce nouveau peptide, en tant que principe actif activateur de la transglutaminase humaine.

L'invention est également relative à l'utilisation cosmétique de ce nouveau peptide, en tant que principe actif, dans une composition, pour renforcer la fonction barrière cutanée et stimuler la régénération et la différenciation épidermique.

L'invention est également relative à ce nouveau peptide utilisé à titre de médicament.

La fonction première de l'épiderme est de constituer une barrière entre l'environnement extérieur et le milieu intérieur. C'est la couche la plus externe de l'épiderme, *le stratum corneum*, qui assure cette fonction. Il est composé de kératinocytes au stade ultime de leur différenciation, les cornéocytes, scellés les uns aux autre par un ciment intercellulaire, à la fois souple et imperméable. On distingue ainsi dans le *stratum corneum* un compartiment cellulaire constitué des cornéocytes et un compartiment extracellulaire principalement constitué de lipides, organisés en structures multilamellaires. Les cornéocytes sont entourés par une membrane spécifique, appelée enveloppe cornée, en grande partie responsable de la résistance, de l'insolubilité et de la souplesse de la peau. L'enveloppe cornée est constituée d'un mélange de protéines structurales reliées entre elles par des liaisons covalentes sous l'action de la transglutaminase. Les principales protéines constitutives de l'enveloppe cornée sont l'envoplakine, la periplakine, l'involucrine, les Small Prolin-Rich proteins (SPR protéins) et la loricrine.

Les transglutaminases (EC 2.3.2.13) sont une famille d'enzymes calcium-dépendantes qui catalysent la formation de ponts peptidiques entre un ε-amino d'un résidu lysine et un γ-carboxamide d'un résidu de glutamine, ces ponts extra et intramoléculaires sont extrêmement résistants à la dégradation (Lorand et al., Nat Rev Mol Cell Biol. Feb;4(2), 2003). Chez l'humain, on a identifié 9 transglutaminases, dont 4 sont exprimées dans la peau.

La transglutaminase-1 (TG1) est exprimée dans les kératinocytes et présente sous forme liée à la membrane.

La transglutaminase-2 (TG2, type 2a ou 2b) n'est présente que dans la couche basale de l'épiderme. Elle est soluble et ne semble pas avoir de rôle dans la formation de l'enveloppe cornée. La TG2 présente la capacité d'induire des liaisons covalentes entre les protéines, mais elle peut également lier le GTP ou le GDP, et se comporter alors comme une G protéine, en particulier lorsque la cellule est en situation d'apoptose ou de nécrose. La TG2 peut également être exportée à la membrane et s'associer avec les intégrines pour augmenter l'adhésion cellulaire, l'étalement des cellules et la migration des cellules sur la fibronectine de la matrice extracellulaire. De part ses fonctions pleïotropiques, la TG2 est entre autre impliquée dans la cicatrisation.

La transglutaminase-3 (TG3) est exprimée dans les follicules pileux et dans les stades ultimes de la différenciation kératinocytaire.

La transglutaminase-5 (TG5) est présente dans les couches supérieures de l'épiderme et joue également un rôle dans les premières étapes de la différenciation épidermique.

Dans l'épiderme, les TG1, TG3 et TG5 sont impliquées dans la formation de l'enveloppe cornée (Lorand et al., Nat Rev Mol Cell Biol. Feb;4(2), 2003).

Les TG ont des substrats très diversifiés. Ainsi, elles sont capables de réticuler les kératines entre elles et avec la filaggrine, ce qui conduit à la stabilisation et la coordination du réseau kératine-filaggrine dans les cellules cornées.

Lors des premiers stades de la différentiation épidermique, les TG améliorent l'ancrage des desmosomes, puis dans la couche granuleuse, ces mêmes TG assurent l'attachement de certains lipides à l'enveloppe cornée et la liaison de la loricrine avec les Small Proline Rich Protein (SPR). Dans les couches granuleuses supérieures, les lipides provenant des corps de golgi sont réticulés par les TG1 et TG5 aux protéines précurseurs de l'enveloppe, déjà partiellement réticulées. Enfin, la phase de desquamation, qui siège au niveau des couches cornées les plus externes, implique des réticulations supplémentaires de la loricrine et d'autres protéines, impliquant la TG1.

Plusieurs études montrent que les trois TG impliquées dans la formation de l'enveloppe cornée dans l'épiderme ont des fonctions différenciées (Candi et al 1995).

Le rôle clé des transglutaminases dans la formation du *stratum corneum,* plus largement dans la différenciation épidermique et en conséquence sur la fonction barrière, est confirmé par certains modèles pathologiques. Ainsi, des souris invalidées pour le gène TG1 homozygotes TG1-/- souffrent de malformations létales du *stratum corneum* et meurent rapidement après la naissance (Matsuki et al, Proc. Nat. Acad. Sci. 95, 1998). D'autre part, chez l'humain, des mutations portées par la TG1 sont responsables de formes plus ou moins graves d'ichthyose (Huber et al. Science 267, 1995).

Au cours du vieillissement cutané, l'intégrité de la barrière cutanée ainsi que ses capacités de réparation s'altèrent. On observe une déficience globale en lipides, aboutissant à une diminution des multicouches lipidiques du compartiment extracellulaire du *stratum corneum*. Ces changements fonctionnels sont en corrélation avec une susceptibilité accrue des peaux âgées aux agressions extérieures (Ghadially R. et al., J Clin Invest., 1995, 95(5) : p. 2281-90).

Indépendamment du vieillissement intrinsèque ou photo-induit, des altérations de la barrière cutanée peuvent se produire lors d'agressions extérieures.

On entend par l'expression « agression extérieure », les agressions que peut produire l'environnement. A titre d'exemple, on peut citer des agressions telles que la pollution, les UV, ou encore les produits à caractère irritant tels que les tensioactifs, les conservateurs ou les parfums, les agressions mécaniques, telles que les abrasions, le rasage ou l'épilation. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. La sécheresse de l'atmosphère est également une cause importante d'agression cutanée. Ces agressions extérieures aboutissent à une altération de la fonction barrière qui se traduit par un inconfort cutané, des phénomènes sensoriels désagréables, tels que des tiraillements ou des démangeaisons, voire des fragilités excessives et des rougeurs.

Les personnes particulièrement concernées par cette altération de la fonction barrière par des agressions extérieures sont les personnes à peau dite "fragile" ou "sensible", c'est-à-dire particulièrement sensibles aux variations de température ou d'humidité et/ou particulièrement réactive vis-à-vis des produits agressifs (cas des peaux de bébé par exemple). Les personnes à peau dite "fragile" regroupent notamment les personnes dont les lipides protecteurs du *stratum corneum* se raréfient, comme c'est le cas pour les personnes âgées et très âgées (au moins 75 ans), les personnes dont la composition des lipides protecteurs du *stratum corneum* est modifiée, comme c'est le cas des personnes diabétiques, ou dialysées, ou atteintes de certaines maladies. Les personnes à peau dite « sensible » possèdent un seuil de réactivité abaissé pouvant être lié à une hyperactivité neurogène. Ces peaux sensibles présenteront des signes cliniques beaucoup plus rapidement et fréquemment que les autres types de peaux.

Une altération de la fonction barrière cutanée peut notamment se traduire par un trouble de l'hydratation, par une perte de souplesse de la peau, par une altération de l'éclat du teint, par l'apparition d'une rugosité sur la peau et plus généralement par l'apparition de signes cutanés du vieillissement.

Il convient alors de chercher à prévenir l'altération ou à rétablir la fonction barrière de l'épiderme. Or, le rôle clé de la transglutaminase en a fait une cible de choix pour renforcer la fonction barrière de l'épiderme. Dans le domaine de la cosmétique, l'activation de la transglutaminase a déjà été décrite dans le but d'améliorer la fonction barrière, en utilisant des extraits végétaux, par exemple des extraits d'*Asiasarum hétérotropoïdes F.*, ou *Asiasarum sieboldi F.*, et d'*Apocynum venetum L*, tels que cités dans la demande de brevet JP2004115451. La demande de brevet US2007134172 décrit l'utilisation de flavonoïdes extraits préférentiellement de *Sidastrum* pour obtenir une meilleure résistance de la peau aux facteurs environnementaux et en particulier le desséchement.

Les inventeurs ont maintenant mis en évidence une activité cosmétique et thérapeutique, notamment dermatologique, de peptides de formule générale (I) :

R₁-(A-A)ₙ-X₁-X₂₋-Arg-Arg-Gly-X₃-X₄-(AA)ₚ-R₂

Il a en particulier été démontré que ces peptides, lorsqu'ils sont appliqués sur la peau, renforcent la fonction barrière de l'épiderme et stimulent la régénération et la différenciation épidermique. Par conséquent, la présente invention concerne des peptides de formule générale (I) correspondant à la séquence (SEQ ID n°5) Arg-Arg-Gly-Gln-NH₂ ainsi que l'utilisation cosmétique dans des compositions comprenant des peptides de formule générale (I) choisies parmi l'une des séquences (SEQ ID n°4) Arg-Arg-Gly-Gln ou (SEQ ID n°5) Arg-Arg-Gly-Gln-NH₂, en tant que principe actif activateur de la transglutaminase humaine, pour protéger la peau et les phanères des agressions extérieures et pour lutter contre les manifestations du vieillissement cutané.

On entend par « peptide ou principe actif activateur de la transglutaminase ou capable d'activer la transglutaminase humaine», tout peptide de formule générale (I) capable d'augmenter l'activité de la transglutaminase soit par l'augmentation de la synthèse protéique de la transglutaminase (par modulation directe ou indirecte de l'expression génique de la transglutaminase), soit par l'augmentation de l'activité enzymatique de la transglutaminase, soit par d'autres processus biologiques tels que la stabilisation de la protéine transglutaminase ou encore la stabilisation des transcrits d'ARN messager.

On entend par peau, l'ensemble des tissus de recouvrement constituant la peau et les muqueuses.

Le terme « phanères » englobe l'ensemble des annexes kératiniques présentes à la surface du corps, en particulier les poils, les cils, les sourcils, les ongles et les cheveux.

On entend par « application topique », le fait d'appliquer ou d'étaler le principe actif, ou une composition le contenant, à la surface de la peau.

On entend par « physiologiquement adapté » que le principe actif, ou une composition le contenant, est approprié pour entrer en contact avec la peau sans provoquer de réactions de toxicité ou d'intolérance.

Ainsi, l'invention a pour objet premier un peptide possédant une séquence qui répond à la formule générale (I) :

R₁-(AA)ₙ-X₁-X₂₋-Arg-Arg-Gly-X₃-X₄-(AA)ₚ-R₂

dans laquelle,
X₁ est l'alanine, la valine ou aucun acide aminé,
X₂ est l'alanine, la valine ou aucun acide aminé,
X₃ est la glutamine ou l'asparagine,
X₄ est la proline, la valine ou aucun acide aminé,
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement de type acyle possédant une chaîne alkyle de C₁ à C₃₀ saturée ou insaturée, pouvant être choisie parmi un groupement acétyle ou un groupement aromatique, le groupement aromatique substituant la fonction amine primaire pouvant être choisi parmi un groupement de type benzoyle, de type tosyle, ou de type benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 4 à 15 résidus d'acides aminés, caractérisé en ce que le peptide est de séquence (SEQ ID n°5) Arg-Arg-Gly-Gln-NH₂.

Les acides aminés, constituant le peptide et désignés sous les termes AA ou X, peuvent être sous configuration isomérique L- et D-. De manière préférentielle, les acides aminés sont sous forme L.

Par « peptide », il faut entendre le peptide naturel ou synthétique tel que décrit ci-dessus, ou au moins l'un de ses fragments, ou au moins l'un de ses dérivés, qu'il soit obtenu par protéolyse ou de manière synthétique, ou encore tout peptide naturel ou synthétique dont la séquence est totalement constituée par la séquence du peptide précédemment décrit.

De façon à améliorer la résistance à la dégradation, il peut être nécessaire d'utiliser une forme protégée du peptide. La forme de protection doit évidemment être une forme biologiquement compatible et doit être compatible avec une utilisation dans le domaine des cosmétiques ou de la pharmacie. De préférence, on utilise pour protéger la fonction amine primaire de l'acide aminé N-terminal, une substitution par un groupement R₁ de type acyle possédant une chaîne alkyle de C₁ à C₃₀ saturée ou insaturée, pouvant être choisie parmi un groupement acétyle ou un groupement aromatique. De préférence on utilise pour protéger la fonction carboxyle de l'acide aminé C-terminal, une substitution par un groupement R₂ de type chaîne alkyle de C₁ à C₃₀, ou un groupement NH2, NHY ou NYY avec Y représentant une chaîne alkyle de C₁ à C₄.

Le peptide peut être protégé au niveau de l'extrémité N-terminale, C-terminale ou au niveau des deux extrémités.

Ainsi, l'invention concerne une composition telle que définie précédemment, caractérisée par le fait que le peptide de séquence SEQ ID n°5 est sous forme protégée ou non.

Le peptide de formule générale (I) de séquence SEQ ID n°4 ou SEQ ID n°5 peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide peut être d'origine naturelle ou synthétique. Préférentiellement, le peptide est obtenu par synthèse chimique.

Selon l'invention, ledit peptide de séquence SEQ ID n°5 peut être utilisé à titre de médicament.

Selon un mode de réalisation avantageux de l'invention, le peptide de séquence SEQ ID n°5 est solubilisé dans un ou plusieurs solvants physiologiquement adaptés, classiquement utilisés par l'homme du métier, comme l'eau, le glycérol, l'éthanol, le propanediol, le propylène glycol, le butylène glycol, le dipropylène glycol, les diglycols éthoxylés ou propoxylés, les polyols cycliques ou tout mélange de ces solvants.

Selon encore un autre mode de réalisation avantageux de l'invention, le peptide de séquence SEQ ID n°5 est solubilisé dans un vecteur cosmétique ou pharmaceutique comme les liposomes, ou adsorbé sur des polymères organiques poudreux, des supports minéraux comme les talcs et bentonites, et plus généralement solubilisé dans, ou fixé sur, tout vecteur physiologiquement adapté.

L'invention a pour second objet une composition cosmétique ou pharmaceutique, et notamment dermatologique, comprenant, dans un milieu physiologiquement adapté, un peptide de formule générale (I) de séquence SEQ ID n°5, en tant que principe actif capable d'activer la transglutaminase humaine. Selon une méthode particulièrement avantageuse de réalisation de l'invention, le peptide est utilisé seul ou en association avec au moins un autre principe actif.

Il est bien évident que l'invention s'adressé aux mammifères en général, et plus particulièrement aux êtres humains.

De manière préférentielle, le peptide de séquence SEQ ID n°5 est plus particulièrement capable d'activer les transglutaminases humaines de type 1, 2a, 2b, 3 ou 5.

Selon un mode de réalisation avantageux de l'invention, le principe actif de séquence SEQ ID n°5 est présent dans les compositions à une concentration comprise entre 0,0005 et 500 ppm (parties par million) environ, et préférentiellement à une concentration comprise entre 0,01 et 5 ppm environ par rapport au poids total de la composition finale.

Cette fourchette de concentrations représente la quantité efficace de principe actif correspondant à la quantité nécessaire pour obtenir le résultat recherché, à savoir, activer la transglutaminase, dans le but de renforcer la fonction barrière cutanée et de stimuler la régénération et la différenciation épidermique.

De manière préférée, la composition se présente sous une forme adaptée à l'application par voie topique comprenant un milieu physiologiquement adapté pour la peau et les phanères. Par « physiologiquement adapté », on entend des milieux qui conviennent à une utilisation en contact avec la peau ou les phanères humains, sans risque de toxicité, d'incompatibilité, d'instabilité, de réponse allergique et autres effets secondaires.

Les compositions destinées à être appliquées sur la peau et les phanères peuvent se présenter sous forme de solution aqueuse ou hydro- alcoolique, d'émulsion eau dans huile ou huile dans eau, de microémulsion, de gel aqueux ou anhydre, de sérum, ou encore de dispersion de vésicules, de patch, de crème, de spray, d'onguent, de pommade, de lotions, de colloïde, de solution, de suspension ou autres.

La composition utilisable selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition cosmétique peut être utilisée notamment dans les traitements mettant en oeuvre une application qui est suivie ou non d'un rinçage, ou encore sous forme de shampooing. Les compositions peuvent aussi être appliquées sur les phanères de teinture ou de mascara à appliquer au pinceau ou au peigne, en particulier sur les cils, les sourcils ou les cheveux, ou encore de soins pour les ongles tels que les vernis.

Il est bien entendu que le principe actif de séquence SEQ ID n°5 peut être utilisé seul ou bien en association avec au moins un autre principe actif, dans une composition cosmétique ou pour la préparation d'une composition pharmaceutique et/ou dermatologique. Avantageusement, les compositions utilisables selon l'invention contiennent en outre divers principes actifs protecteurs ou antivieillissement, destinés, notamment, à la prévention et/ou au traitement des désordres liés au vieillissement.

On peut citer, de manière non limitative, les classes d'ingrédients suivantes : d'autres agents actifs peptidiques, des extraits de végétaux, des agents cicatrisants, anti-âge, antirides, apaisants, anti-radicalaire, anti-UV, des agents stimulant la synthèse de macromolécules dermiques ou le métabolisme énergétique, des agents hydratants, anti-bactériens, anti-fongiques, anti-inflammatoires, anesthésiques, des agents modulants la différenciation, la pigmentation ou la dépigmentation cutanée, des agents stimulants la pousse des ongles ou des cheveux.... Préférentiellement, on utilisera un agent anti-radicalaire ou antioxydant, ou un agent stimulant la synthèse de macromolécules dermiques, ou encore un agent stimulant le métabolisme énergétique.

En outre, des additifs tels que des agents épaississants, émulsifiants, humectants, émollients, des parfums, des antioxydants, des agents filmogènes, chélatants, séquestrants, conditionnants, etc., peuvent être ajoutés à la composition.

Les compositions pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions sont destinées à une administration par voie topique cutanée. Ces compositions doivent donc contenir un milieu physiologiquement adapté, c'est-à-dire compatible avec la peau et les phanères, et couvrent toutes les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, épaississants, diluants, tensioactifs, antioxydants, colorants, conservateurs, parfums.

Selon une autre forme de l'invention, les compositions seront appropriées à une administration orale pour un usage pharmaceutique. Ainsi, les compositions pourront notamment se présenter sous forme de comprimés, capsules, gélules, pâtes à mâcher, poudres à consommer telles quelles ou à mélanger extemporanément avec un liquide, sirops, gels, et toute autre forme connue de l'homme du métier. Elles contiendront des excipients de formulation appropriés, tels que colorants, édulcorants, aromatisants, agents de charge, liants, conservateurs.

Ces compositions pourront notamment se présenter sous forme d'une solution aqueuse, hydro-alcoolique ou huileuse ; d'une émulsion huile-dans-eau, eau-dans-huile ou émulsions multiples; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de manière à ne pas nuire aux propriétés avantageuses recherchées de la composition. Ces adjuvants peuvent, par exemple, correspondre à 0,01 à 20 % du poids total de la composition. Lorsque la composition est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

L'invention a pour troisième objet l'utilisation cosmétique d'une composition comprenant le peptide de formule générale (I) :

R₁-(AA)ₙ-X₁-X₂--Arg-Arg-Gly-X₃-X₄-(AA)ₚ-R₂

dans laquelle,
X₁ est l'alanine, la valine ou aucun acide aminé,
X₂ est l'alanine, la valine ou aucun acide aminé,
X₃ est la glutamine ou l'asparagine,
X₄ est la proline, la valine ou aucun acide aminé,
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement de type acyle possédant une chaîne alkyle de C₁ à C₃₀ saturée ou insaturée, pouvant être choisie parmi un groupement acétyle ou un groupement aromatique, le groupement aromatique substituant la fonction amine primaire pouvant être choisi parmi un groupement de type benzoyle, de type tosyle, ou de type benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 4 à 15 résidus d'acides aminés, choisie parmi l'une des séquences suivantes :
   (SEQ ID n°4) Arg-Arg-Gly-Gln
   (SEQ ID n°5) Arg-Arg-Gly-Gln-NH2,
   en tant que principe actif, pour renforcer la fonction barrière cutanée et de stimuler la régénération et la différenciation épidermique.

On entend par «renforcer la fonction barrière cutanée et stimuler la régénération et la différenciation épidermique», l'amélioration de la structure de la couche cornée, l'augmentation des signes de régénérations cellulaires, tels que la densité des couches basales épidermiques et la vitesse de migration des fibroblastes, et l'augmentation de l'expression des marqueurs de différenciation kératinocytaire.

L'invention a pour quatrième objet l'utilisation cosmétique d'une composition comprenant le peptide de formule générale (I) de séquence SEQ ID n°4 ou SEQ ID n°5, en tant que principe actif, pour lutter de manière préventive et/ou curative contre les signes du vieillissement cutané, et plus particulièrement le vieillissement photo-induit (photo-vieillissement). Par manifestations cutanées du vieillissement, on entend toutes modifications de l'aspect extérieur de la peau et des phanères dues au vieillissement comme, par exemple, les rugosités superficielles de la couche cornée, les rides et ridules, mais également toute modification interne de la peau qui ne se traduit pas systématiquement par un aspect extérieur modifié comme, par exemple, l'amincissement du derme ou toute autre dégradation interne de la peau consécutive à une exposition aux rayonnements ultraviolets (UV).

L'invention a pour cinquième objet l'utilisation cosmétique d'une composition comprenant le peptide de formule générale (I) de séquence SEQ ID n°4 ou SEQ ID n°5, en tant que principe actif, pour protéger la peau et les phanères contre tout type d'agressions extérieures.

En particulier, l'invention a pour objet l'utilisation cosmétique d'une composition comprenant une quantité efficace de peptide de séquence SEQ ID n°4 ou SEQ ID n°5 pour prévenir ou traiter les dommages causés à la peau et aux phanères par des traitements mécaniques tels que le rasage ou l'épilation.

En particulier, l'invention a pour objet l'utilisation cosmétique d'une composition comprenant une quantité efficace de peptide de séquence SEQ ID n°4 ou SEQ ID n°5, pour prévenir ou traiter les dommages causés à la peau et aux phanères par des conditions climatiques extrêmes ou des variations brutales de températures et d'hygrométrie.

En particulier, l'invention a pour objet l'utilisation cosmétique d'une composition comprenant une quantité efficace de peptide de séquence SEQ ID n°4 ou SEQ ID n°5, pour prévenir ou traiter les dommages causés à la peau et aux phanères par des expositions aux rayonnements ultraviolets (UV).

On applique topiquement sur la peau ou les phanères à traiter une composition contenant une quantité efficace de principe actif pour prévenir ou traiter les signes cutanés du vieillissement ou protéger la peau et les phanères contre les agressions extérieures, en particulier, pour protéger la peau et les phanères contre les agressions dues aux rayonnements UV.

Un dernier objet de l'invention concerne l'utilisation du peptide de formule générale (I)

R₁ -(AA)ₙ-X₁-X₂--Arg-Arg-Gly-X₃-X₄-(AA)ₚ-R₂

dans laquelle,
X₁ est l'alanine, la valine ou aucun acide aminé,
X₂ est l'alanine, la valine ou aucun acide aminé,
X₃ est la glutamine ou l'asparagine,
X₄ est la proline, la valine ou aucun acide aminé,
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement de type acyle possédant une chaîne alkyle de C₁ à C₃₀ saturée ou insaturée, pouvant être choisie parmi un groupement acétyle ou un groupement aromatique, le groupement aromatique substituant la fonction amine primaire pouvant être choisi parmi un groupement de type benzoyle, de type tosyle, ou de type benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄.
ladite séquence de formule générale (I) étant constituée de 4 à 15 résidus d'acides aminés, choisie parmi l'une des séquences suivantes :
   (SEQ ID n°4) Arg-Arg-Gly-Gln
   (SEQ ID n°5) Arg-Arg-Gly-Gln-NH2,
   en tant que principe actif, utilisé seul ou en association avec au moins un autre principe actif, dans une composition pharmaceutique destinée à prévenir ou traiter les pathologies caractérisées par une altération de la fonction barrière, telles que les peaux hypersensibles, irritées, ou réactives et l'eczéma atopique.

D'autres avantages et caractéristiques de l'invention apparaîtront mieux à la lecture des exemples donnés à titre illustratif et non limitatif.

### Liste des figures

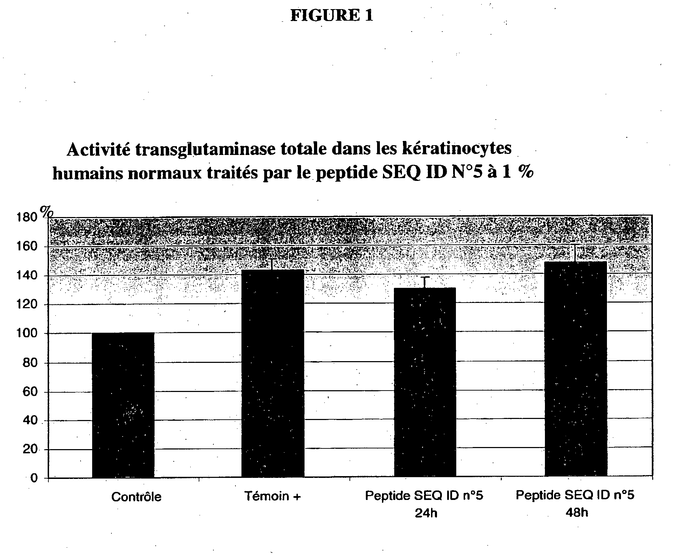

Figure 1 : Graphe représentant les résultats du dosage de l'activité transglutaminase totale dans les kératinocytes humains normaux traités par le peptide SEQ ID N°5 à 1 %.

### Exemple 1 : Mise en évidence de l'augmentation de l'activité enzymatique globale des transglutaminases par le peptide SEQ ID n°5

Le but de cette étude est de déterminer l'influence du peptide SEQ ID n°5 sur l'activité totale des transglutaminases dans les kératinocytes humains normaux (KHN). Pour cela, l'activité enzymatique totale des transglutaminases est dosée par spectrophotométrie à l'aide d'un substrat donneur d'acides aminés marqué à la fluorescéine.

Protocole : Des KHN sont ensemencés dans des plaques 96 puits noirs. Après 4 jours de culture, les KHN sont traités avec une solution à 1 % d'une solution mère à 50 ppm du peptide SEQ ID n°5 pendant 24 ou 48 heures (le principe actif est rajouté toutes les 24 heures). Un contrôle positif est réalisé, par traitement des cellules à l'EGCG à 20 µg/ml (épigallocatéchine gallate, le principal polyphénol du thé vert). Le substrat utilisé est la cadavérine marquée à la fluorescéine (Invitrogen A10466) diluée à 100 µM dans du milieu de culture. Le substrat est incubé pendant 2 heures avec les cellules à raison de 200 µl/puits). Les cellules sont ensuite rincées deux fois dans du tampon HBSS et fixées par un mélange acide acétique-éthanol-eau (1:49:50) pendant 20 min. Après deux rinçages dans de l'éthanol puis trois rinçages dans du tampon HBSS, 100 µl de PBS sont ajoutés dans chaque puits et une lecture au spectrophotomètre à des longueurs d'onde d'excitation de 485 nm et d'émission de 530 nm est réalisée. Dans ces conditions, l'activité transglutaminase est proportionnelle à la quantité de fluorescence émise (exprimée en unités de fluorescence), rapportée à la quantité totale de protéines présente dans chaque puits, préalablement dosée par la technique BCA.

Résultats: Les résultats sont exprimés en pourcentage par rapport au contrôle non traité et présentés dans la Figure 1. En présence de 0,5 ppm de peptide SEQ ID n°5, l'activité enzymatique est augmentée de 30 % après 24 heures et 47,7 % après 48 heures.

Conclusion : Le peptide SEQ ID n°5 augmente significativement l'activité enzymatique totale des transglutaminases dans les kératinocytes humains normaux.

### Exemple 2 : Mise en évidence de l'effet activateur du peptide SEQ ID n°5 sur l'expression des TG1, TG2, TG3 et TG5

Le but de cette étude est de déterminer l'influence du peptide SEQ ID n°5 sur l'expression des différentes transglutaminases exprimées dans la peau humaine. Pour cela des marquages spécifiques par immunofluorescence ont été réalisés sur culture de kératinocytes humains normaux (KHN) et sur biopsie de peau. Des marquages par immunofluorescence ont également été réalisés sur des cultures de fibroblastes humains normaux spécifiquement pour la TG2, exprimée dans ce type de cellules.

Protocole des immunomarquages sur kératinocytes humains normaux en culture : Des KHN en culture sont traités avec une solution à 1 % d'une solution mère à 50 ppm du peptide SEQ ID n°5 pendant 24 heures. Pour l'immunomarquage par l'anticorps anti TG1, les cellules sont lavées et fixées au paraformaldéhyde à 3,7% pendant 10 minutes. Les cellules sont ensuite incubées en présence d'un anticorps spécifique anti TG1 (Clinisciences BT-621, mouse monoclonal), puis d'un anticorps secondaire adapté, couplé à un marqueur fluorescent. Pour les autres immunomarquages, les cellules sont lavées et fixées au méthanol froid pendant 1 minute. Les cellules sont ensuite incubées en présence d'un anticorps spécifique ; anti-TG2 (Abcam ab2972, rabbit polyclonal), anti TG3 (Abcam ab53236, mouse monoclonal) ou anti TG5 (Abcam ab26992, rabbit polyclonal) Après montage dans un milieu *ad hoc*, les lames sont observées au microscope à épifluorescence (Nikon Eclipse E 80i microscope).

Protocole des immunomarquages sur fibroblastes humains normaux en culture : Des fibroblastes humains dermiques sont traités et immunomarqués à l'aide d'un anticorps anti TG2 selon le même protocole que pour les KHN.

Protocole des immunomarquage sur biopsies de peau : Des biopsies de peau humaine sont mises en culture à l'interface air/liquide. Une solution à 1 %, d'une solution mère à 50 ppm de peptide SEQ ID n°5 est appliquée topiquement durant 24 heures.

Pour les marquages de la TG1 et de la TG2, les biopsies de peau sont ensuite incluses dans de la résine et congelées dans l'azote. Des coupes d'environ 6 µm sont ensuite réalisées au cryostat. L'immunomarquage est réalisé à l'aide d'un anticorps spécifique ; anti TG1 (Clinisciences BT-621, mouse monoclonal) ou anti-TG2 (Abcam ab2972, rabbit polyclonal) puis d'un anticorps secondaire adapté, couplé à un marqueur fluorescent. Les coupes de peau sont alors examinées au microscope à Epi-fluorescence (Nikon Eclipse E 80i microscope).

Pour les marquages de la TG3, les biopsies de peau sont incluses dans de la paraffine et des coupes histologiques de 3 µm d'épaisseur sont effectuées. Les lames sont déparaffinées, hydratées puis soumises à un immunomarquage par un anticorps dirigé contre la TG3 (Abcam ab53236, mouse monoclonal) puis d'un anticorps secondaire adapté, couplé à un marqueur fluorescent. Les coupes de peau sont alors examinées au microscope à Epi-fluorescence (Nikon Eclipse E 80i microscope).

Résultats : Dans toutes les conditions testées, on observe une fluorescence plus intense dans les cultures et sur les coupes de peaux traitées par le peptide SEQ ID n°5 à 0,5 ppm que dans les conditions contrôle, non traité.

Conclusions : Le peptide SEQ ID n°5 à 0,5 ppm stimule l'expression des TG1, TG2, TG3 et TG5 dans les kératinocytes humains normaux en culture, ainsi que l'expression de la TG2 dans les fibroblastes humains.

Le peptide SEQ ID n°5 à 0,5 ppm stimule l'expression des TG1, TG2 et TG3 dans des biopsies de peaux cultivées *ex vivo*.

### Exemple 3 : Mise en évidence de l'effet activateur du peptide SEQ ID n°5 sur la différenciation épidermique

Le but de cette étude est de déterminer l'influence du peptide SEQ ID n°5 sur la différenciation épidermique. Pour cela, l'expression des principaux marqueurs de la différenciation épidermique, exprimés spécifiquement dans les kératinocytes des couches suprabasales, a été étudiée. Les marqueurs testés sont la transglutaminase 1, les pankératines, la filaggrine, l'involucrine et la loricrine. D'autre part, la filaggrine, l'involucrine et la loricrine sont des précurseurs de l'enveloppe cornée et des substrats des transglutaminases.

Protocole des immunomarquages sur kératinocytes humains normaux en culture : Des KHN en culture sont traités avec une solution à 1 % d'une solution mère à 50 ppm du peptide SEQ ID n°5 pendant 24 heures. Les cellules sont ensuite lavées et fixées au paraformaldéhyde à 3,7% pendant 10 minutes. Après démasquage des sites spécifiques, les cellules sont incubées en présence d'un anticorps spécifique dirigé contre la TG1 (Clinisciences BT-621, mouse monoclonal), ou contre la loricrine (Abcam ab24722, rabbit polyclonal), ou l'involucrine (Novocastra NCL-INV, mouse monoclonal, clone SY5), puis incubées en présence d'un anticorps secondaire adapté, couplé à un marqueur fluorescent. Pour une plus grande facilité d'observation, les noyaux des cellules peuvent être contre-colorés par le DAPI (4',6' Di Amidino-2-Phényl Indole), une molécule fluorescente bleue capable de se lier fortement à l'ADN). Après montage dans un milieu *ad hoc*, les lames sont observées au microscope à épifluorescence (Nikon Eclipse E 80i microscope).

Protocole des immunomarquages sur biopsies de peau : Des biopsies de peau humaine sont mises en culture à l'interface air/liquide. Une solution à 1 % d'une solution mère à 50 ppm de peptide SEQ ID n°5 est appliquée topiquement durant 24 heures. Les biopsies de peau sont ensuite incluses dans la paraffine et des coupes histologiques de 3 µm d'épaisseur sont effectuées. Les lames sont déparaffinées, hydratées puis soumises à un immunomarquage par un anticorps dirigé contre la TG1 (Clinisciences BT-621, mouse monoclonal) ou les cyto-pankératines (Novocastra (NCL-CK10, mouse monoclonal), ou la loricrine (Abcam ab24722, rabbit polyclonal), ou l'involucrine (Novocastra NCL-INV, clone SY5, mouse monoclonal) ou la filaggrine (Tebu Santa Cruz sc-58761, mouse monoclonal). Pour une plus grande facilité d'observation, les noyaux des cellules peuvent être contre-colorés par le DAPI (4',6' Di Amidino-2-Phényl Indole, une molécule fluorescente bleue capable de se lier fortement à l'ADN). Un anticorps secondaire adapté, couplé à un marqueur fluorescent, est ensuite utilisé. Après montage dans un milieu *ad hoc*, les lames sont observées au microscope à épifluorescence (Nikon Eclipse E 80i microscope).

Protocole des immunotransferts : Des KHN en culture sont traités avec une solution à 1 % d'une solution mère à 50 ppm du peptide SEQ ID n°5 pendant 24 heures. Les cellules sont ensuite rincées puis détachées du support par grattage dans un tampon RIPA en présence d'un cocktail d'inhibiteurs de protéases (Thermo Scientific, Rockford, USA). Les cellules lysées sont centrifugées à 4°C à 10000 rpm pendant 20 minutes et les surnageants collectés. Les échantillons sont ensuite standardisés par dosage des protéines par le kit BCA (Pierce, France). Les échantillons sont mélangés puis soumis à une électrophorèse sur gel NuPAGE 3-8% Tris-acetate, dans un tampon de migration NuPAGE Tris-acetate, puis transférés sur membrane de nitrocellulose à l'aide d'un dispositif de transfert (Invitrogen, Paisley, UK). Les membranes sont saturées dans du TBS-lait 5 %, 2 heures à température ambiante, puis incubées à 4°C toute la nuit avec un anticorps primaire dirigé contre la TG1 (Clinisciences BT-621, mouse monoclonal) ou contre l'involucrine (Novocastra NCL-INV, clone SY5, mouse monoclonal) ou contre la loricrine (Abcam ab24722, rabbit polyclonal). Après lavage par du tampon TBS-Tween 0,05%, la membrane est incubée avec un anticorps secondaire adapté, couplé à la péroxydase. Les transferts sont ensuite développés à l'aide d'un substrat chimioluminescent (SuperSignal West Dura Extended Duration Substrate, Pierce, Brebiere, France). Les bandes de protéines spécifiques ainsi révélées sont quantifiées à l'aide d'un analyseur d'image Chemi-Imager technology (Alpha Innotech Corporation).

Résultats : Dans toutes les conditions testées par immunofluorescence, on observe une fluorescence plus intense dans les cultures et sur les coupes de peaux traitées par le peptide SEQ ID n°5 à 0,5 ppm que dans les conditions contrôle, non traité. Sur les coupes de peau *ex vivo*, on note une différenciation épidermique avec présence d'une couche cornée plus épaisse.
L'analyse quantitative des immunotransferts permet d'évaluer l'augmentation de l'expression des marqueurs testés. L'augmentation de la TG1 est de 20 %, de l'involucrine est de 30 % et de la loricrine est de 16 %.

Conclusions : Le peptide SEQ ID n°5 stimule l'expression des pankératines, de l'involucrine, de la filaggrine, de la loricrine, ainsi que de la TG1 dans les kératinocytes humains normaux. Le peptide SEQ ID n°5 à 0,5 ppm améliore également la différenciation épidermique et en particulier la morphologie de la couche cornée.

### Exemple 4 : Mise en évidence de l'effet protecteur vis-à-vis des agressions extérieures du peptide SEQ ID n°5

Le but de cette étude est de déterminer l'effet protecteur sur la peau du peptide selon l'invention vis-à-vis d'agressions extérieures. Pour cela, un modèle *ex vivo* d'agression sévère de la barrière cutanée a été utilisé.

Protocole : Les biopsies de peau humaine sont soumises à une agression provoquée par des arrachements successifs de strates de la couche cornée à l'aide de ruban adhésif (technique dite de « tape stripping »). L'étape d'arrachement est répétée 20 fois de suite sur la même zone. Les biopsies de peau humaine « strippées » sont ensuite mises en culture et traitées par le peptide SEQ ID n°5 à 0,5 ppm et 1,5 ppm, selon le protocole de l'exemple 2, pendant 48 heures. Les biopsies de peau sont ensuite incluses dans de la paraffine et des coupes histologiques de 3 µm d'épaisseur sont réalisées. Les coupes sont déposées sur des lames Superfrost Plus (Menzel Gläser, Thermo Scientific), puis déparaffinées dans le xylène et réhydratées dans une série de solutions alcool-eau. Les coupes sont ensuite colorées par de l'hématoxyline à 50 % pendant 3 min, rincées, puis colorées à l'éosine 60 %, pendant 3 min et rincées à l'eau. Les coupes sont déshydratées, montées dans l'Eukitt et examinées en microscopie optique.

Résultats : Les coupes histologiques de peau traitée par le peptide SEQ ID n°5 à 0,5 et 1,5 ppm montrent une néo-synthèse plus importante des couches cornées. L'ensemble des couches épidermiques présente moins de cellules vacuolées ainsi qu'une plus grande densité cellulaire.

Conclusion : Le peptide SEQ ID n°5 améliore la reconstruction de l'épiderme agressé.

### Exemple 5 : Mise en évidence de l'effet régénérant du peptide SEQ ID n°5

Le but de cette étude est de déterminer l'effet régénérant du peptide SEQ ID n°5 sur les fibroblastes dermiques et sur l'épiderme.

Protocole d'utilisation du modèle *in vitro* de régénération fibroblastique Ibidi : Le modèle de cicatrisation *In vitro* Ibidi a été utilisé (Biovalley, Marne la Vallée, France). Des fibroblastes humains sont ensemencés dans deux compartiments séparés d'un insert Ibidi puis celui-ci est déposé dans une boîte de culture. A confluence, l'insert est enlevé créant ainsi une zone acellulaire de 400 µm de large entre les deux tapis cellulaires. Le peptide dilué à 1 % et 3 % à partir d'une solution mère à 50 ppm du peptide SEQ ID n°5 est alors ajouté au milieu de culture, et le traitement est effectué pendant 48 heures avec renouvellement du principe actif toutes les 24 heures. Des observations en microscopie à contraste de phase (Olympus CK40 microscope, x5, connecté à un appareil photo Olympus E-510) ont été réalisées à différents temps (0 à 48 heures) pendant le processus de migration.

Protocole d'étude de la régénération épidermique : Des biopsies de peau humaine sont mis en culture à l'interface air/liquide. Une solution à 1 %, d'une solution mère à 50 ppm de peptide SEQ ID n°5 est appliquée topiquement, puis les échantillons sont incubés durant 24 heures. Les biopsies de peau sont ensuite incluses dans de la paraffine et des coupes histologiques de 3 µm d'épaisseur sont réalisées. Les coupes sont déposées sur des lames Superfrost Plus (Menzel Gläser, Thermo Scientific), puis déparaffinées dans le xylène et réhydratées dans une série de solutions alcool-eau. Les coupes sont ensuite colorées par de l'hématoxyline à 50 % pendant 3 min, rincées, puis colorées à l'éosine 60 %, pendant 3 min et rincées à l'eau. Les coupes sont ensuite déshydratées, montées dans l'Eukitt et examinées en microscopie optique.

### Résultats :

i) L'envahissement par les fibroblastes de la zone acellulaire est plus rapide lorsque les cellules sont traitées par le peptide SEQ ID n°5 à 0,5 et 1,5 ppm, comparé aux conditions contrôle. L'effet est dose-dépendant.
ii) Les coupes histologiques d'épiderme traités par le peptide SEQ ID n°5 montrent une néo-synthèse de la couche cornée, qui apparait plus épaisse et mieux hydratée. On observe également une plus grande densité des cellules de la couche basale, qui apparaissent mieux orientées dans l'axe vertical et plus homogènes.
Conclusions : Le peptide SEQ ID n°5 assure une régénération des fibroblastes dermiques ainsi que de l'épiderme. Il induit une plus grande cohésion des couches cornées.

### Exemple 6 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n° 5 | | 3 ppm |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2-Crème anti-âge :

| ***Noms commerciaux*** | ***Noms INCI*** | **% *massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n°5 | | 0,5 ppm |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 3 -Crème protectrice de jour :

| ***Noms commerciaux*** | ***Noms INCI*** | **% *massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C 10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n°5 | | 5 ppm |

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Peptide de formule générale (I) :
R₁-(AA)ₙ-X₁-X₂₋-Arg-Arg-Gly-X₃-X₄-(AA)ₚ-R₂
dans laquelle,
X₁ est l'alanine, la valine ou aucun acide aminé,
X₂ est l'alanine, la valine ou aucun acide aminé,
X₃ est la glutamine ou l'asparagine,
X₄ est la proline, la valine ou aucun acide aminé,
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement de type acyle possédant une chaîne alkyle de C₁ à C₃₀ saturée ou insaturée, pouvant être choisie parmi un groupement acétyle ou un groupement aromatique. Le groupement aromatique substituant la fonction amine primaire peut être choisi parmi un groupement de type benzoyle, de type tosyle, ou de type benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH₂, NHY ou NYY avec Y représentant une chaîne alkyle de C₁ à C₄,
ladite séquence de formule générale (I) étant constituée de 4 à 15 résidus d'acides aminés **caractérisé en ce qu'**il correspond à la séquence (SEQ ID n°5) Arg-Arg-Gly-Gln-NH₂.

2. Peptide selon la revendication précédente, **caractérisé en ce qu'**il est utilisé à titre de médicament.

3. Peptide selon l'une des revendications précédentes **caractérisé en ce qu'**il est solubilisé dans un ou plusieurs solvants physiologiquement adaptés choisi parmi l'eau, le glycérol, l'éthanol, le propanediol, le propylène glycol, le butylène glycol, le dipropylène glycol, les diglycols éthoxylés ou propoxylés, les polyols cycliques ou tout mélange de ces solvants.

4. Composition cosmétique ou pharmaceutique **caractérisée en ce qu'**elle comprend, dans un milieu physiologiquement adapté, le peptide tel que défini dans l'une quelconque des revendications 1 à 3, en tant que principe actif activateur de la transglutaminase humaine, utilisé seul ou en association avec au moins un autre principe actif.

5. Composition cosmétique ou pharmaceutique selon la revendication 4 **caractérisée en ce que** le principe actif est capable d'activer les transglutaminases humaines de type 1, 2a, 2b, 3 ou 5.

6. Composition cosmétique ou pharmaceutique selon l'une des revendications 4 ou 5, **caractérisée en ce que** ledit peptide est présent à une concentration comprise entre 0,0005 et 500 ppm environ.

7. Composition cosmétique ou pharmaceutique selon la revendication 6 **caractérisée en ce que** ledit peptide est présent à une concentration comprise entre 0,01 et 5 ppm.

8. Composition cosmétique ou pharmaceutique selon l'une des revendications 4 à 7, **caractérisée en ce qu'**elle est destinée à une administration par voie topique.

9. Composition cosmétique ou pharmaceutique selon l'une des revendications 4 à 8, **caractérisée en ce qu'**elle contient, en outre, au moins un autre principe actif ayant des propriétés protectrices ou des propriétés d'antivieillissement choisi parmi les principes actifs antioxydants, ou stimulant la synthèse des macromolécules dermiques, ou stimulant le métabolisme énergétique.

10. Utilisation cosmétique d'une composition comprenant un peptide de formule générale (I) :
R₁-(AA)ₙ-X₁-X₂₋-Arg-Arg-Gly-X₃-X₄-(AA)ₚ-R₂
dans laquelle,
X₁ est l'alanine, la valine ou aucun acide aminé,
X₂ est l'alanine, la valine ou aucun acide aminé,
X₃ est la glutamine ou l'asparagine,
X₄ est la proline, la valine ou aucun acide aminé,
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement de type acyle possédant une chaîne alkyle de C₁ à C₃₀ saturée ou insaturée, pouvant être choisie parmi un groupement acétyle ou un groupement aromatique. Le groupement aromatique substituant la fonction amine primaire peut être choisi parmi un groupement de type benzoyle, de type tosyle, ou de type benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 4 à 15 résidus d'acides aminés choisie parmi l'une des séquences suivantes :
(SEQ ID n°4) Arg-Arg-Gly-Gln,
(SEQ ID n°5) Arg-Arg-Gly-Gln-NH₂,
pour renforcer la fonction barrière de l'épiderme et stimuler la régénération et la différenciation épidermique.

11. Utilisation cosmétique d'une composition selon la revendication 10 pour prévenir et lutter contre les signes cutanés du vieillissement et du photo-vieillissement.

12. Utilisation cosmétique d'une composition selon la revendication 10 pour protéger la peau et les phanères contre tout type d'agressions extérieures.

13. Peptide de formule générale (I) :
R₁-(AA)ₙ-X₁-X₂₋-Arg-Arg-Gly-X₃-X₄-(AA)ₚ-R₂
dans laquelle,
X₁ est l'alanine, la valine ou aucun acide aminé,
X₂ est l'alanine, la valine ou aucun acide aminé,
X₃ est la glutamine ou l'asparagine,
X₄ est la proline, la valine ou aucun acide aminé,
AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement de type acyle possédant une chaîne alkyle de C₁ à C₃₀ saturée ou insaturée, pouvant être choisie parmi un groupement acétyle ou un groupement aromatique. Le groupement aromatique substituant la fonction amine primaire peut être choisi parmi un groupement de type benzoyle, de type tosyle, ou de type benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 4 à 15 résidus d'acides aminés choisie parmi l'une des séquences suivantes :
(SEQ ID n°4) Arg-Arg-Gly-Gln,
(SEQ ID n°5) Arg-Arg-Gly-Gln-NH₂,
en tant que principe actif, utilisé seul ou en association avec au moins un autre principe actif, dans une composition pharmaceutique destinée à prévenir ou traiter les pathologies **caractérisées par** une altération de la fonction barrière choisies parmi les peaux hypersensibles, irritées, ou réactives et l'eczéma atopique.

## Patentansprüche

1. Peptid der allgemeinen Formel (I):
R₁-(AA)ₙ-X₁-X₂-Arg-Arg-Gly-X₃-X₄- (AA)ₚ-R₂,
in der
X₁ Alanin, Valin oder keine Aminosäure ist,
X₂ Alanin, Valin oder keine Aminosäure ist,
X₃ Glutamin oder Asparagin ist,
X₄ Prolin, Valin oder keine Aminosäure ist,
AA für eine beliebige Aminosäure oder eines ihrer Derivate steht und n und p ganze Zahlen zwischen 0 und 4 sind,
R₁ für die primäre Aminofunktion der N-terminalen Aminosäure steht, die frei oder durch eine Gruppe des Acyltyps substituiert ist, die über eine gesättigte oder
ungesättigte C₁- bis C₃₀-Alkylkette verfügt und die aus einer Acetylgruppe oder einer aromatischen Gruppe ausgewählt sein kann, wobei die aromatische Gruppe, die die primäre Aminofunktion substituiert, aus einer Gruppe des Benzoyltyps, des Tosyltyps oder des Benzyloxycarbonyltyps ausgewählt sein kann,
R₂ für die Hydroxylgruppe der Carboxylfunktion der C-terminalen Aminosäure steht, die frei oder durch eine Gruppe substituiert ist, die aus einer C₁- bis C₃₀-Alkylkette oder einer NH₂-, NHY- oder NYY-Gruppe, wobei Y für eine C₁- bis C₄-Alkylkette steht, ausgewählt sein kann,
wobei die besagte Sequenz der allgemeinen Formel (I) aus 4 bis 15 Aminosäureresten besteht, **dadurch gekennzeichnet, dass** sie der Sequenz (SEQ ID Nr. 5) Arg-Arg-Gly-Gln-NH₂ entspricht.

2. Peptid nach dem vorhergehenden Anspruch, **dadurch gekennzeichnet, dass** es als Arzneimittel verwendet wird.

3. Peptid nach einem der vorhergehenden Ansprüche, **dadurch gekennzeichnet, dass** es in einem oder mehreren physiologisch angepassten Lösungsmitteln solubilisiert wird, das bzw. die aus Wasser, Glycerin, Ethanol, Propandiol, Propylenglykol, Butylenglykol, Dipropylenglykol, ethoxylierten oder propoxylierten Diglykolen, cyclischen Polyolen oder jeglichem Gemisch dieser Lösungsmittel ausgewählt ist bzw. sind.

4. Kosmetische oder pharmazeutische Zusammensetzung, **dadurch gekennzeichnet, dass** sie in einem physiologisch angepassten Medium das wie in einem der Ansprüche 1 bis 3 definierte Peptid als Wirkstoff, der humane Transglutaminase aktiviert, umfasst, der allein oder in Verbindung mit mindestens einem anderen Wirkstoff verwendet wird.

5. Kosmetische oder pharmazeutische Zusammensetzung nach Anspruch 4, **dadurch gekennzeichnet, dass** der Wirkstoff humane Transglutaminasen des Typs 1, 2a, 2b, 3 oder 5 aktivieren kann.

6. Kosmetische oder pharmazeutische Zusammensetzung nach einem der Ansprüche 4 oder 5, **dadurch gekennzeichnet, dass** das besagte Peptid in einer Konzentration zwischen ungefähr 0,0005 und 500 ppm vorliegt.

7. Kosmetische oder pharmazeutische Zusammensetzung nach Anspruch 6, **dadurch gekennzeichnet, dass** das besagte Peptid in einer Konzentration zwischen 0,01 und 5 ppm vorliegt.

8. Kosmetische oder pharmazeutische Zusammensetzung nach einem der Ansprüche 4 bis 7, **dadurch gekennzeichnet, dass** sie für eine topische Verabreichung vorgesehen ist.

9. Kosmetische oder pharmazeutische Zusammensetzung nach einem der Ansprüche 4 bis 8, **dadurch gekennzeichnet, dass** sie außerdem mindestens einen anderen Wirkstoff mit Schutzeigenschaften oder Anti-Aging-Eigenschaften enthält, der aus antioxidierenden Wirkstoffen oder Wirkstoffen, die die Synthese von dermalen Makromolekülen stimulieren, oder Wirkstoffen, die den Energiestoffwechsel stimulieren, ausgewählt ist.

10. Kosmetische Verwendung einer Zusammensetzung, die ein Peptid der allgemeinen Formel (I) umfasst:
R₁-(AA)ₙ-X₁-X₂-Arg-Arg-Gly-X₃-X₄- (AA)ₚ-R₂,
in der
X₁ Alanin, Valin oder keine Aminosäure ist,
X₂ Alanin, Valin oder keine Aminosäure ist,
X₃ Glutamin oder Asparagin ist,
X₄ Prolin, Valin oder keine Aminosäure ist,
AA für eine beliebige Aminosäure oder eines ihrer Derivate steht und n und p ganze Zahlen zwischen 0 und 4 sind,
R₁ für die primäre Aminofunktion der N-terminalen Aminosäure steht, die frei oder durch eine Gruppe des Acyltyps substituiert ist, die über eine gesättigte oder ungesättigte C₁- bis C₃₀-Alkylkette verfügt und die aus einer Acetylgruppe oder einer aromatischen Gruppe ausgewählt sein kann, wobei die aromatische Gruppe, die die primäre Aminofunktion substituiert, aus einer Gruppe des Benzoyltyps, des Tosyltyps oder des Benzyloxycarbonyltyps ausgewählt sein kann,
R₂ für die Hydroxylgruppe der Carboxylfunktion der C-terminalen Aminosäure steht, die frei oder durch eine Gruppe substituiert ist, die aus einer C₁- bis C₃₀-Alkylkette oder einer NH₂-, NHY- oder NYY-Gruppe, wobei Y für eine C₁- bis C₄-Alkylkette steht, ausgewählt sein kann,
wobei die besagte Sequenz der allgemeinen Formel (I) aus 4 bis 15 Aminosäureresten besteht und aus einer der folgenden Sequenzen ausgewählt ist:
(SEQ ID Nr. 4) Arg-Arg-Gly-Gln,
(SEQ ID Nr. 5) Arg-Arg-Gly-Gln-NH₂,
zum Verstärken der Barrierefunktion der Epidermis und zum Stimulieren der epidermalen Differenzierung.

11. Kosmetische Verwendung einer Zusammensetzung nach Anspruch 10 zum Verhindern und Bekämpfen von Zeichen der Alterung und der Lichtalterung der Haut.

12. Kosmetische Verwendung einer Zusammensetzung nach Anspruch 10 zum Schützen der Haut und der Hautanhangsgebilde vor jeglicher Art von äußeren Einflüssen.

13. Peptid der allgemeinen Formel (I):
R₁-(AA)ₙ-X₁-X₂-Arg-Arg-Gly-X₃-X₄- (AA)ₚ-R₂,
in der
X₁ Alanin, Valin oder keine Aminosäure ist,
X₂ Alanin, Valin oder keine Aminosäure ist,
X₃ Glutamin oder Asparagin ist,
X₄ Prolin, Valin oder keine Aminosäure ist,
AA für eine beliebige Aminosäure oder eines ihrer Derivate steht und n und p ganze Zahlen zwischen 0 und 4 sind,
R₁ für die primäre Aminofunktion der N-terminalen Aminosäure steht, die frei oder durch eine Gruppe des Acyltyps substituiert ist, die über eine gesättigte oder ungesättigte C₁- bis C₃₀-Alkylkette verfügt und die aus einer Acetylgruppe oder einer aromatischen Gruppe ausgewählt sein kann, wobei die aromatische Gruppe, die die primäre Aminofunktion substituiert, aus einer Gruppe des Benzoyltyps, des Tosyltyps oder des Benzyloxycarbonyltyps ausgewählt sein kann,
R₂ für die Hydroxylgruppe der Carboxylfunktion der C-terminalen Aminosäure steht, die frei oder durch eine Gruppe substituiert ist, die aus einer C₁- bis C₃₀-Alkylkette oder einer NH₂-, NHY- oder NYY-Gruppe, wobei Y für eine C₁- bis C₄-Alkylkette steht, ausgewählt sein kann,
wobei die besagte Sequenz der allgemeinen Formel (I) aus 4 bis 15 Aminosäureresten besteht und aus einer der folgenden Sequenzen ausgewählt ist:
(SEQ ID Nr. 4) Arg-Arg-Gly-Gln,
(SEQ ID Nr. 5) Arg-Arg-Gly-Gln-NH₂,
als Wirkstoff, der allein oder in Verbindung mit mindestens einem anderen Wirkstoff verwendet wird, in einer pharmazeutischen Zusammensetzung, die zum Verhindern oder Behandeln von Pathologien vorgesehen ist, die durch eine Veränderung der Barrierefunktion gekennzeichnet sind und aus überempfindlicher, gereizter oder reaktiver Haut und atopischem Ekzem ausgewählt sind.

## Claims

1. Peptide of general formula (I):
R₁-(AA)ₙ-X₁-X₂-Arg-Arg-Gly-X₃-X₄-(AA)ₚ-R₂
wherein,
X₁ is alanine, valine or no amino acid,
X₂ is alanine, valine or no amino acid,
X₃ is glutamine or asparagine,
X₄ is proline, valine or no amino acid,
AA represents any amino acid, or one of its derivatives,
and n and p are integers between 0 and 4,
R₁ represents the primary amine function of the N-terminal amino acid, free or substituted by an acyl-type group having a saturated or unsaturated C₁ to C₃₀ alkyl chain, capable of being chosen from an acetyl group or an aromatic group. The aromatic group substituting the primary amine function may be chosen from a benzoyl-, tosyl-, or benzyloxycarbonyl-type group.
R₂ represents the hydroxyl group of the carboxyl function of the C-terminal amino acid, free or substituted by a group capable of being chosen from a C₁ to C₃₀ alkyl chain, or an NH₂, NHY or NYY group with Y representing a C₁ to C₄ alkyl chain,
said sequence of general formula (I) consisting of 4 to 15 amino acid residues, **characterized in that** it corresponds to sequence (SEQ ID no. 5) Arg-Arg-Gly-Gln-NH₂.

2. Peptide according to the previous claim, **characterized in that** it is used as a drug.

3. Peptide according to one of the previous claims, **characterized in that** it is solubilized in one or more physiologically adapted solvents chosen from water, glycerol, ethanol, propanediol, propylene glycol, butylene glycol, dipropylene glycol, ethoxylated or propoxylated diglycols, cyclic polyols or any mixture of said solvents.

4. Cosmetic or pharmaceutical composition **characterized in that** it includes, in a physiologically adapted medium, the peptide as defined in any one of claims 1 to 3, as an active principle activating human transglutaminase, used alone or in association with at least one other active principle.

5. Cosmetic or pharmaceutical composition according to claim 4, **characterized in that** the active principle is capable of activating human transglutaminases of type 1, 2a, 2b, 3 or 5.

6. Cosmetic or pharmaceutical composition according to one of claims 4 or 5, **characterized in that** said peptide is present in a concentration of between around 0.0005 and 500 ppm.

7. Cosmetic or pharmaceutical composition according to claim 6, **characterized in that** said peptide is present in a concentration of between 0.01 and 5 ppm.

8. Cosmetic or pharmaceutical composition according to one of claims 4 to 7, **characterized in that** it is intended for topical administration.

9. Cosmetic or pharmaceutical composition according to one of claims 4 to 8, **characterized in that** it also contains at least one active principle having protective properties or anti-aging properties chosen from the antioxidant active principles, or stimulating the synthesis of dermal macromolecules, or stimulating energy metabolism.

10. Cosmetic use of a composition including a peptide of general formula (I):
R₁-(AA)ₙ-X₁-X₂-Arg-Arg-Gly-X₃-X₄-(AA)ₚ-R₂
wherein,
X₁ is alanine, valine or no amino acid,
X₂ is alanine, valine or no amino acid,
X₃ is glutamine or asparagine,
X₄ is proline, valine or no amino acid,
AA represents any amino acid, or one of its derivatives,
and n and p are integers between 0 and 4,
R₁ represents the primary amine function of the N-terminal amino acid, free or substituted by an acyl-type group having a saturated or unsaturated C₁ to C₃₀ alkyl chain, capable of being chosen from an acetyl group or an aromatic group. The aromatic group substituting the primary amine function may be chosen from a benzoyl-, tosyl-, or benzyloxycarbonyl-type group.
R₂ represents the hydroxyl group of the carboxyl function of the C-terminal amino acid, free or substituted by a group capable of being chosen from a C₁ to C₃₀ alkyl chain, or an NH₂, NHY or NYY group with Y representing a C₁ to C₄ alkyl chain,
said sequence of general formula (I) consisting of 4 to 15 amino acid residues chosen from one of the following sequences:
(SEQ ID No. 4) Arg-Arg-Gly-Gln,
(SEQ ID no. 5) Arg-Arg-Gly-Gln-NH₂,
in order to reinforce the barrier function of the epidermis and stimulate epidermal differentiation and regeneration.

11. Cosmetic use of a composition according to claim 10 for preventing and combatting the signs of skin aging and photo-aging.

12. Cosmetic use of a composition according to claim 10 for protecting the skin and skin appendages from any type of external stress.

13. Peptide of general formula (I):
R₁-(AA)ₙ-X₁-X₂-Arg-Arg-Gly-X₃-X₄- (AA)ₚ-R₂
wherein,
X₁ is alanine, valine or no amino acid,
X₂ is alanine, valine or no amino acid,
X₃ is glutamine or asparagine,
X₄ is proline, valine or no amino acid,
AA represents any amino acid, or one of its derivatives, and n and p are integers between 0 and 4,
R₁ represents the primary amine function of the N-terminal amino acid, free or substituted by an acyl-type group having a saturated or unsaturated C₁ to C₃₀ alkyl chain, capable of being chosen from an acetyl group or an aromatic group. The aromatic group substituting the primary amine function may be chosen from a benzoyl-, tosyl-, or benzyloxycarbonyl-type group.
R₂ represents the hydroxyl group of the carboxyl function of the C-terminal amino acid, free or substituted by a group capable of being chosen from a C₁ to C₃₀ alkyl chain, or an NH₂, NHY or NYY group with Y representing a C₁ to C₄ alkyl chain,
said sequence of general formula (I) consisting of 4 to 15 amino acid residues chosen from one of the following sequences:
(SEQ ID No. 4) Arg-Arg-Gly-Gln,
(SEQ ID no. 5) Arg-Arg-Gly-Gln-NH₂,
as an active principle, used alone or in combination with at least one other active principle, in a pharmaceutical composition intended to prevent or treat pathologies **characterized by** an alteration in the barrier function chosen from hypersensitive, irritated or reactive skin and atopic eczema.


### SEQUENCE LISTING

<110> ISP Investments INC.
<120> PEPTIDE ACTIVATEUR DE LA TRANSGLUTAMINASE ET COMPOSITION COSMETIQUE OU PHARMACEUTIQUE LE CONTENANT
<130> Bv PCT 09-130
<150> 0904218
   <151> 2009-09-04
<160> 8
<170> PatentIn version 3.3
<210> 1
   <211> 10
   <212> PRT
   <213> Synthetic peptide
<400> 1
   Val Val Arg Arg Gly Gln Pro Phe Trp Leu
   1 5 10
<210> 2
   <211> 8
   <212> PRT
   <213> synthetic peptide
<220>
   <221> MOD_RES
   <222> (8)..(8)
   <223> AMIDATION
<400> 2
   Val Ala Arg Arg Gly Gln Pro Phe
   1 5
<210> 3
   <211> 7
   <212> PRT
   <213> Synthetic peptide
<400> 3
   Ala Ala Arg Arg Gly Asn Pro
   1 5
<210> 4
   <211> 4
   <212> PRT
   <213> Synthetic peptide
<400> 4
   Arg Arg Gly Gln
   1
<210> 5
   <211> 4
   <212> PRT
   <213> Synthetic peptide
<220>
   <221> MOD_RES
   <222> (4)..(4)
   <223> AMIDATION
<400> 5
   Arg Arg Gly Gln
   1
<210> 6
   <211> 6
   <212> PRT
   <213> Synthetic peptide
<400> 6
   Ala Ala Arg Arg Gly Asn
   1               5
<210> 7
   <211> 6
   <212> PRT
   <213> Synthetic peptide
<220>
   <221> MOD_RES
   <222> (6)..(6)
   <223> AMIDATION
<400> 7
   Val Val Arg Arg Gly Gln
   1               5
<210> 8
   <211> 6
   <212> PRT
   <213> Synthetic peptide
<400> 8
   Ala Val Arg Arg Gly Asn
   1               5